# EUROPÄISCHE PATENTSCHRIFT

(11) **EP 2 081 973 B1**
(45) Veröffentlichungstag und Bekanntmachung des Hinweises auf die Patenterteilung: **05.05.2010**
(21) Anmeldenummer: 07820713.1
(22) Anmeldetag: 28.09.2007
(51) Int. Cl.: C08G 18/66, C08G 18/79, C08G 18/12, B01D 63/02

(54) **VERGUSSMASSEN AUF BASIS VON POLYURETHAN**
CASTING COMPOUNDS BASED ON POLYURETHANE
MASSE DE SCELLEMENT À BASE DE POLYURÉTHANNE

(30) Priorität: 04.10.2006 EP 06121741
(43) Veröffentlichungstag der Anmeldung: 29.07.2009
(73) Patentinhaber: BASF SE, 67056 Ludwigshafen (DE)
(72) Erfinder: REESE, Hans-Jürgen, 49401 Damme (DE); FRITZ, Ralf, 49143 Bissendorf-Schledehausen (DE); LUKAT, Gunther, 49163 Bohmte (DE); SCHMIDT, Hans Ulrich, 49090 Osnabrück (DE)
(86) Internationale Anmeldenummer: PCT/EP2007/060326
(87) Internationale Veröffentlichungsnummer: WO 2008/040687

(56) Entgegenhaltungen:
- EP-A- 1 088 841
- EP-A- 1 090 941
- US-A- 5 451 615

## Beschreibung

Die Erfindung betrifft eine Polyolmischung, die als Komponenten
a1) mindestens ein fettbasiertes Polyol,
a2) mindestens ein Polyetherol mit einem zahlenmittleren Molekulargewicht von 500 bis 2000 g/mol und
a3) ein oder mehrere Vernetzer mit einem zahlenmittleren Molekulargewicht von 90 bis 400 g/mol,
enthält, wobei
x) das zahlenmittlere Molekulargewicht der Komponente a1) um höchstens 400 g/mol vom zahlenmittleren Molekulargewicht der Komponente a2) abweicht
und xi) die OH-Funktionalität der Komponenten a1) und a2) um nicht mehr als 0.5 voneinander abweicht,
wobei die Polyolmischung, bezogen das Gesamtgewicht von a1), a2) und a3), von 45 bis 65 Gew.-% der Komponente a1), von 34 bis 54 Gew.-% der Komponente a2) und von 1 bis 21 Gew.-% der Komponente a3) enthält,

Diese Polyolmischungen können zur Herstellung von Vergussmassen auf Basis von Polyurethan eingesetzt werden. Darüber hinaus betrifft die Erfindung Vergussmassen auf Basis der eingangs beschriebenen Polyolmischungen (A) und mindestens eines modifizierten Isocyanats (B), sowie die Verwendung der Vergussmassen als Einbettungswerkstoff. Weiterhin betrifft die Erfindung ein Verfahren zur Herstellung der eingangs beschriebenen Vergussmassen. Außerdem betrifft die Erfindung Dialysefilter, welche die eingangs beschriebenen Vergussmassen umfassen. Schließlich betrifft die Erfindung die Kombination aus getrennt vorliegenden Komponenten (A) und (B) zur gemeinsamen Anwendung.

Bevorzugte Ausführungsformen sind den Ansprüchen und der Beschreibung zu entnehmen. Kombinationen bevorzugter Ausführungsformen verlassen nicht den Rahmen dieser Erfindung.

Vergussmassen auf Basis von Polyurethan (PU) sind an sich bekannt und werden z.B. zusammenfassend beschrieben im Kunststoff-Handbuch "Polyurethane", Band 7, 3. Auflage, 1993, Seiten 438-454, herausgegeben von Dr. G. Oertel, im Carl Hanser Verlag, München, Wien.

Die Verwendung von Vergussmassen auf Basis von Polyurethan zur Herstellung von Formteilen für medizinisch-technische Geräte, insbesondere als Einbettwerkstoff für Hohlfasern in Dialysatoren, ist ebenfalls an sich bekannt und besitzt unter anderem aufgrund der einfachen Handhabung von PU-Vergussmassen sowie deren geringe Schrumpfung während des Aushärtungsprozesses Vorteile.

EP-0 538 673 beschreibt transparente, heissdampfsterilisierbare, nicht zytotoxische Vergussmassen auf Basis von Polyurethan, die durch Umsetzung von modifizierten Diisocyanaten mit einer Polyolkomponente erhältlich sind. Als geeignete Bestandteile der Polyolkomponente werden niedermolekulare Polyetherpolyole mit einer mittleren Funktionalität von 3 bis 8 und einer Hydroxylzahl von 200 bis 1000 mg KOH/g sowie entweder Rizinusöl oder Polyetherpolyole mit einer Funktionalität von 2 bis 3 und einer Hydroxylzahl von 90 bis 200 mg KOH/g offenbart. Kombinationen aus beiden letztgenannten Komponenten werden zwar nicht ausgeschlossen, jedoch werden keine Angaben über besonders geeignete, insbesondere niedrigviskose Polyolmischungen gemacht.

EP-A-1 090 941 beschreibt ebenfalls transparente, heissdampfsterilisierbare, nicht zytotoxische Vergussmassen auf Basis von Polyurethan. Die in dieser Anmeldung genannten Polyurethansysteme basieren auf einer modifizierten Isocyanat-Komponente mit spezifischer Viskosität sowie auf einer Polyolkomponente, die u.a. auch Rizinusöl im Gemisch mit niedermolekularen Polyetherolen mit einer Funktionalität von 3 bis 8 und einer Hydroxylzahl von 200 bis 1000 mg KOH/g enthalten kann.

DE-31 49 527 A1 beschreibt Polyurethanmassen für Hohlfaserdialysatoren, welche Polyisocyanate und lagerstabile Polyolmischungen aus hauptsächlich Rizinusöl enthalten. Die lagerstabile Polyolmischung enthält außerdem noch üblicherweise mit Rizinusöl nicht verträgliche, möglichst niedermolekulare hydroxylgruppenhaltige Verbindungen, z. B. niedermolekulare Alkohole, sowie deren partielle Ester mit langkettigen Carbonsäuren als Lösungsvermittler.

Ein Nachteil der im Stand der Technik beschriebenen Polyolmischungen ist die hohe Viskosität nach Mischung der Reaktivkomponenten. Als Konsequenz hieraus ist das zeiteffiziente Vergießen insbesondere von Dialysefiltern mit einer hohen Faserzahl problematisch oder unmöglich.

Als Alternative schlägt die EP-A-1 582 544 Vergusssysteme auf Basis von Polyurethan vor, welche eine niedrige Viskosität aufweisen. Die vorgeschlagenen Zusammensetzungen basieren auf dem Einsatz eines besonderen Diolgemisches zur Herstellung eines niederviskosen Polyisocyanat-Präpolymers, wobei bevorzugt Propylenglykole mit unterschiedlichem Molekulargewicht zum Einsatz kommen. Als Polyolkomponente werden Polyetheralkohole und/oder Polyesteralkohole vorgeschlagen. Die in EP-A-1 582 544 vorgeschlagenen Vergusssysteme sind jedoch hinsichtlich der Stabilität bei der Nasssterilisation noch verbesserungswürdig.

Eine Aufgabe der vorliegenden Erfindung war es, Polyolmischungen bereitzustellen, die eine niedrige Viskosität unmittelbar nach Mischung mit einer Isocyanatkomponente (im folgenden Mischviskosität genannt) aufweisen und so das Vergießen in Dialysefiltern mit einer hohen Faserzahl von bevorzugt mehr als 12000 Fasern pro Filter zu ermöglichen. Gleichzeitig sollten die mit den erfindungsgemäßen Polyolmischungen hergestellten Vergussmassen eine hohe Stabilität bei der Nasssterilisation aufweisen. Ein Ziel war demzufolge, Vergussmassen mit einer lediglich geringfügigen Aufnahme von Wasser bei hohen Temperaturen und einer hohen Resistenz gegenüber Desinfektionsmitteln, insbesondere eine geringfügige Desorption von Peressigsäure nach der Nasssterilisation bereitzustellen. Darüber hinaus sollten die Vergussmassen eine geringe Feinstaubbildung beim Verschneiden sowie eine gute Verschneidbarkeit über einen langen Zeitraum aufweisen. Schließlich sollten günstige Haftungseigenschaften zwischen der Vergussmasse und dem Gehäuse von medizinisch-technischen Artikeln erreicht werden. Die Vergussmassen sollten nach erfolgter Aushärtung in Kontakt mit wässrigen Medien außerdem keine Desorption toxischer Verbindungen zeigen und transparent sein.

Es wurde gefunden, dass sich die oben genannten positiven Eigenschaften, insbesondere eine geringe Wasseraufnahme, eine hohe Resistenz gegenüber Desinfektionsmitteln und gute Verarbeitungseigenschaften nach dem Aushärten einerseits sowie eine niedrige Mischviskosität andererseits gleichzeitig erreichen lassen, indem die erfindungsgemäßen Polyolmischungen eingesetzt werden. Es wurde außerdem gefunden, dass die erfindungsgemäßen Vergussmassen die beschriebenen vorzüglichen Eigenschaften bei der Nasssterilisation und bei der Verarbeitung haben.

Eine weitere Aufgabe bestand schließlich darin, ein Verfahren zur Herstellung von Vergussmassen und Dialysefiltern, insbesondere solche mit einer hohen Faserzahl, bereitzustellen, bei dem ein zeiteffizientes Vergießen selbst komplexer Formen ohne die Bildung von Hohlräumen möglich ist.

### Polyolmischungen

Erfindungsgemäß enthält die Polyolmischung mindestens ein fettbasiertes Polyol a1) und mindestens ein Polyetherol a2) mit einem zahlenmittleren Molekulargewicht von 500 bis 2000 g/mol, wobei das zahlenmittlere Molekulargewicht der beiden Polyole um höchstens 400 g/mol und die OH-Funktionalität der beiden Polyole um höchstens 0.5 voneinander abweicht und mindestens einen Vernetzer a3) mit einem zahlenmittleren Molekulargewicht von 90 bis 400 g/mol, wobei die Polyolmischung, bezogen auf das Gesamtgewicht von a1), a2) und a3) von 45 bis 65 Gew.-% der Komponente a1), von 34 bis 54 Gew.-% der Komponente a2) und von 1 bis 21 Gew.-% der Komponente a3) enthält, wobei die Summe aus a1), a2) und a3) 100 Gew.-% ergibt.

Unter OH-Funktionalität ist die Zahl an alkoholischen, acylierbaren OH-Gruppen pro Molekül zu verstehen. Falls die betreffende Komponente aus einer Verbindung mit definierter Molekularstruktur besteht, ergibt sich die Funktionalität aus der Zahl der OH-Gruppen pro Molekül. Wird eine Verbindung durch Ethoxylierung oder Propoxylierung eines Startermoleküls hergestellt, ergibt sich die OH-Funktionalität aus der Anzahl der reaktiven funktionellen Gruppen, beispielsweise OH-Gruppen, pro Startermolekül. Falls Mischungen aus Verbindungen mit unterschiedlicher OH-Funktionalität zum Einsatz kommen, ergibt sich die OH-Funktionalität aus dem zahlengewichteten Mittel der OH-Funktionalität der einzelnen Verbindungen.

Alle in dieser Erfindung genannten Molekulargewichte bezeichnen das zahlenmittlere Molekulargewicht. Das Molekulargewicht einer Mischung oder einer Komponente ergibt sich dabei aus den zahlengewichteten Molekulargewichten der enthaltenen Verbindungen. Unter einem zahlenmittleren Molekulargewicht soll im folgenden derjenige Wert verstanden werden, der mittels Gelpermeationschromatographie an einem Ultrastyragelsäulensystem mit Tetrahydrofuran (THF) als Laufmittel und einem RI-Detektor bei 35°C bestimmt wird.

Unter einem Polyol ist eine Verbindung zu verstehen, die pro Molekül mindestens zwei gegenüber Isocyanat-Gruppen reaktive Wasserstoffatome enthält. Bevorzugt stammen die gegenüber Isocyanatgruppen reaktiven H-Atome von Hydroxylgruppen.

Bevorzugt enthält die Polyolmischung die Komponenten a1) und a2) in einem Gewichtsverhältnis von a1) zu a2) von 8 zu 2 bis 2 zu 8. Besonders bevorzugt ist ein Gewichtsverhältnis der Komponenten a1) zu a2) von 7 zu 3 bis 4 zu 6, beispielsweise von 6,5 zu 3,5 bis 4,5 zu 5.5.

Besonders bevorzugt beträgt der Anteil von a3) in der Polyolmischung von 5 bis 10 Gew.-%, jeweils bezogen auf die Polyolmischung.

Die erfindungsgemäße Polyolmischung weist bevorzugt eine Viskosität von bis zu 1500 mPa·s auf; bevorzugt ist eine Viskosität von bis zu 1000 mPa·s und besonders bevorzugt ist eine Viskosität von bis zu 700 mPa·s. Ganz besonders bevorzugt ist schließlich eine Viskosität der Polyolmischung von bis zu 600 mPa·s. Grundsätzlich ist eine möglichst geringe Viskosität der Polyolmischung erwünscht, da eine niedrige Viskosität beim späteren Herstellen einer Vergussmasse auf Basis von Polyurethan zu einer niedrigen Mischviskosität führt. Aufgrund der erfindungsgemäßen Zusammensetzung der Polyolmischung ergibt sich jedoch eine praktische untere Grenze für die Viskosität. Beispielsweise kann die Viskosität der Polyolmischung im Bereich von 200 mPa·s oder höher liegen, insbesondere im Bereich von 250 bis 600 mPa·s.

Die Viskosität kann beispielsweise mittels eines Rotationsviskosimeters bestimmt werden. Alle in dieser Erfindung genannten Viskositäten beziehen sich auf die Bestimmung nach DIN 53018 bei einer Temperatur von 25°C mit einem Rotationsviskosimeter in Platte/Kegel-Messgeometrie.

Erfindungsgemäß enthält die Komponente a1) mindestens ein fettbasiertes Polyol. Bevorzugt besitzt die Komponente a1) eine OH-Funktionalität von mindestens 2. Als Komponente a1) geeignet sind somit unter anderem Mischungen aus fettbasierten Polyolen, die jeweils eine OH-Funktionalität von mindestens 2 aufweisen, oder Mischungen aus fettbasierten Polyolen, wobei sich eine OH-Funktionalität der Komponente a1) von mindestens 2 ergibt.

Die OH-Funktionalität der Komponente a1) liegt bevorzugt im Bereich von 2 bis 3. Besonders bevorzugt weist die Komponente a1) eine OH-Funktionalität von 2,3 bis 3 auf und ganz besonders bevorzugt von 2,6 bis 3.

Unter fettbasiertem Polyol soll eine Verbindung verstanden werden, welche auf einem Fett, einem Öl, einer Fettsäure oder einem Fettsäurederivat basiert. Ein fettbasiertes Polyol kann ein Fett, ein Öl, eine Fettsäure oder ein Fettsäurederivat sein oder aus den vorgenannten Verbindungen durch physikalische oder chemische Modifikation erhalten werden. Fettbasierte Polyole nach oben genannter Definition sind dem Fachmann an sich bekannt oder können nach an sich bekannten Methoden erhalten werden.

Als fettbasiertes Polyol kommen beispielsweise pflanzliche Öle oder deren Derivate in Betracht. Pflanzliche Öle können in ihrer Zusammensetzung schwanken und in unterschiedlichen Reinheitsgraden vorkommen. Bevorzugt im Sinne dieser Erfindung sind pflanzliche Öle, die den Bestimmungen des Deutschen Arzneibuches (DAB) genügen. Ganz besonders bevorzugt enthält die Komponente a1) mindestens ein fettbasiertes Polyol, das ein pflanzliches Öl ist, welches DAB-10 entspricht.

Als fettbasiertes Polyol können außerdem allgemein bekannte Fettsäuren, bevorzugt natürliche Fettsäuren, besonders bevorzugt pflanzliche Fettsäuren, insbesondere ungesättigte pflanzliche Fettsäuren, als auch deren Derivate wie die Ester mit Mono- und/oder Dialkoholen verwendet werden, sofern die weiter unten diskutierten Eigenschaften hinsichtlich Molekulargewicht und OH-Funktionalität erfüllt sind.

Als fettbasiertes Polyol können jedoch beispielsweise auch ringgeöffnete epoxidierte oder oxidierte Fettsäureverbindungen und/oder Addukte von Fettsäureverbindungen und Alkylenoxiden eingesetzt werden. Bevorzugt sind hydroxylierte Fettsäuren und/oder hydroxylierte Fettsäurederivate, die durch die vorgenannten Verfahren erhältlich sind.

Die Addukte von OH-funktionellen fettbasierten Verbindungen, beispielsweise Rizinusöl oder hydroxylierte pflanzliche Öle, und Alkylenoxiden können durch allgemein bekannte Alkoxylierung der Verbindungen mit beispielsweise Ethylenoxid, Propylenoxid und/oder Butylenoxid bei Temperaturen von 80 bis 130 °C und Drücken von 0,1 bis 1 MPa, gegebenenfalls in Gegenwart von üblichen Katalysatoren wie Alkalihydroxiden oder Alkalialkoholaten hergestellt werden.

Als fettbasiertes Polyol können des weiteren auch hydroxylierte Fettsäureverbindungen auf der Basis von Rapsöl, Sojaöl, Rüböl, Olivenöl und/oder Sonnenblumenöl und/oder solche auf der Basis von Öl- und/oder Linolsäure eingesetzt werden. Als fettbasiertes Polyol sind insbesondere Polyole geeignet, die auf hydroxyliertem Sojaöl basieren.

Bevorzugt wird als fettbasiertes Polyol jedoch ein pflanzliches Öl ohne chemische Modifikation eingesetzt. Besonders bevorzugt ist Rizinusöl. Insbesondere bevorzugt als fettbasiertes Polyol ist Rizinusöl, das den Bestimmungen des Deutschen Arzneibuches gemäß DAB 10 genügt.

Bevorzugt sind außerdem Triglyceride von Fettsäuren, die eine OH-Funktionalität von 2 bis 3 aufweisen. Besonders bevorzugt sind das Triglycerid von Ricinolsäure, gegebenenfalls im Gemisch mit Triglyceriden, die noch weitere natürliche Fettsäuren enthalten, beispielsweise Linolsäure und/oder Palmitinsäure.

Die Komponente a1) weist bevorzugt einen geringen Wassergehalt, beispielsweise kleiner 0,2 Gew.-% auf. Bevorzugt ist ein Wassergehalt der Komponente a1) von weniger als 0,1 Gew.-%. Wird als Komponente a1) ein natürliches Öl, beispielsweise Rizinusöl, eingesetzt, dann erfolgt üblicherweise vor dem Einsatz eine Reinigung, die insbesondere die Entfernung von Schwebstoffen und eine Entwässerung einschließen kann. Von Schwebstoffen befreite natürliche Öle mit oben genanntem Wassergehalt sind als Komponente a1) besonders geeignet.

Das Polyol kann neben seinem Molekulargewicht auch mittels seiner Hydroxylzahl charakterisiert werden. Wie dem Fachmann hinlänglich bekannt, ist eine exakte Umrechnung von Molekulargewicht in Hydroxylzahl lediglich bei bekannter OH-Funktionalität möglich. Die Hydroxylzahl der Komponente a1) beträgt bevorzugt 50 bis 350 mg KOH/g, besonders bevorzugt 100 bis 300 mg KOH/g, und ganz besonders bevorzugt 100 bis 200 mg KOH/g.

Die Hydroxylzahl einer Verbindung gibt an, welche Menge an Kaliumhydroxid in Milligramm der von 1 g der Verbindung bei der Acetylierung gebundenen Essigsäure äquivalent ist. Die Hydroxylzahl ist ein Maß für die Konzentration von Hydroxylgruppen in einer Polymerkette. Die Bestimmung der Hydroxylzahl ist in der DIN 53240 beschrieben, auf die sich die in dieser Anmeldung angegebenen Hydroxylzahlen beziehen.

In Komponente a1) eingesetzt werden bevorzugt fettbasierte Polyole mit einem zahlenmittleren Molekulargewicht von 500 bis 2000 g/mol. Besonders bevorzugt werden fettbasierte Polyole mit einem zahlenmittleren Molekulargewicht von 700 bis 1400 g/mol eingesetzt, ganz besonders bevorzugt von 800 bis 1100 g/mol. Die Komponente a1) hat bevorzugt ein zahlenmittleres Molekulargewicht von 500 bis 200 g/mol, besonders bevorzugt von 700 bis 1400 g/mol, und ganz besonders bevorzugt von 800 bis 1100 g/mol.

Als Komponente a1) insbesondere bevorzugt werden fettbasierte Polyole oder Mischungen aus mehreren fettbasierten Polyolen, wobei das zahlenmittlere Molekulargewicht der Komponente a1) von 700 bis 1400 g/mol und die OH-Funktionalität von 2 bis 3 beträgt; ganz besonders bevorzugt ist ein zahlenmittleres Molekulargewicht der Komponente a1) von 800 bis 1100 g/mol und eine OH-Funktionalität von 2,6 bis 3.

Erfindungsgemäß enthält die Komponente a2) mindestens ein Polyetherol mit einem zahlenmittleren Molekulargewicht von 500 bis 2000 g/mol. Das zahlenmittlere Molekulargewicht der Komponente a2) liegt bevorzugt im Bereich von 700 bis 1400 g/mol und besonders bevorzugt von 800 bis 1100 g/mol.

Vorzugsweise weist die Komponente a2) eine OH-Funktionalität von 2 bis 4 und besonders bevorzugt von 2,5 bis 3,5 auf. Ganz besonders bevorzugt hat die Komponente a2) eine OH-Funktionalität von 3.

Der Gehalt an Alkaliionen in der Komponente a2) kann herstellungsbedingt in einem weiten Bereich variieren. Üblicherweise enthält die Komponente a2) von 0 bis 200 ppm Alkaliionen. Bevorzugt weist die Komponente a2) einen geringen Gehalt an Alkaliionen auf, beispielsweise nicht mehr als 20 ppm. Besonders bevorzugt weist die Komponente a2) einen Alkaliionengehalt von nicht mehr als 10 ppm auf.

Polyetherole mit den vorgenannten Eigenschaften sind dem Fachmann an sich bekannt oder können mittels an sich bekannter Verfahren hergestellt werden, beispielsweise durch anionische Polymerisation mit Alkalihydroxiden, wie Natrium- oder Kaliumhydroxid oder Alkalialkoholaten, wie Natriummethylat, Natrium- oder Kaliumethylat oder Kaliumisopropylat als Katalysatoren und unter Zusatz mindestens eines Startermoleküls, das 2 bis 4 reaktive Wasserstoffatome gebunden enthält, oder durch kationische Polymerisation mit Lewis-Säuren, wie Antimonpentachlorid, Borfluorid-Etherat u.a. oder Bleicherde als Katalysatoren aus einem oder mehreren Alkylenoxiden, ausgewählt aus Propylenoxid (PO) und Ethylenoxid (EO), hergestellt werden.

Falls unterschiedliche Alkylenoxide in ein Polyetherol der Komponente a2) eingebaut werden, können diese einzeln, alternierend nacheinander oder als Mischungen verwendet werden. Der Einsatz eines EO/PO-Gemisches führt zu einem Polyetherol mit statistischer PO/EO-Einheiten-Verteilung. Es ist möglich, zunächst ein PO/EO-Gemisch einzusetzen und dann vor Abbruch der Polymerisation nur noch PO oder EO zu verwenden, um ein Polyetherpolyol mit PO- bzw. EO-Endcap zu erhalten.

Als Startermoleküle zur Herstellung der Polyetherole der Komponente a2) kommen beispielsweise in Betracht: Wasser, organische Dicarbonsäuren, Diamine, wie z.B. gegebenenfalls mono- und dialkylsubstituiertes Ethylendiamin, Diethylentriamin, Triethylentetramin, 1,3-Propylendiamin, und/oder 1,3- bzw. 1,4-Butylendiamin. Als Startermoleküle kommen ferner in Betracht: Alkanolamine, wie z.B. Ethanolamin, N-Methyl- und N-Ethylethanolamin, Dialkanolamine, wie z.B. Diethanolamin, N-Methyl- und N-Ethyldiethanolamin und Trialkanolamine wie z.B. Triethanolamin und Ammoniak. Weiterhin können als Startermoleküle zwei-, drei- oder vierwertige Alkohole, wie E-thandiol, Propandiol-1,2 und -1,3, Diethylenglykol, Dipropylenglykol, Butandiol-1,4, Hexandiol-1,6, Glycerin und/oder Pentaerythrit eingesetzt werden.

Bevorzugt sind Startermoleküle oder Mischungen aus Startermolekülen, die zu Polyetherolen mit den oben genannten bevorzugten OH-Funktionalitäten führen.

Bevorzugt enthält die Komponente a2) mindestens ein Polyetherol auf Basis von propoxyliertem Trimethylolpropan oder propoxyliertem Glycerin oder Mischungen dieser Verbindungen. Besonders bevorzugt sind dabei propoxyliertes Trimethylolpropan und/oder propoxyliertes Glycerin mit einem zahlenmittleren Molekulargewicht von 700 bis 1400 g/mol, ganz besonders bevorzugt von 800 bis 1100 g/mol.

Die Komponente a2) kann grundsätzlich eine oder mehrere der vorgenannten Polyetherole enthalten. Der Fachmann wählt Mischungen derart, dass sich die vorgenannten Eigenschaften der Komponente a2) hinsichtlich OH-Funktionalität und Molekulargewicht einstellen.

Erfindungsgemäß weicht das zahlenmittlere Molekulargewicht der Komponente a1) um höchstens 400 g/mol vom zahlenmittleren Molekulargewicht der Komponente a2) und die OH-Funktionalität der Komponente a1) von der OH-Funktionalität der Komponente a2) um höchstens 0,5 ab. Bevorzugt beträgt die Abweichung des zahlenmittleren Molekulargewichts höchstens 200 g/mol, ganz besonders bevorzugt höchstens 100 g/mol.

Bevorzugt beträgt die Abweichung der OH-Funktionalitäten nicht mehr als 0,4 und ganz besonders bevorzugt nicht mehr als 0,3.

Besonders bevorzugt weicht das zahlenmittlere Molekulargewicht der Komponente a1) um höchstens 200 g/mol vom zahlenmittleren Molekulargewicht der Komponente a2) und die OH-Funktionalität der Komponente a1) um höchstens 0,4 von der OH-Funktionalität der Komponente a2) ab. Ganz besonders bevorzugt weicht das zahlenmittlere Molekulargewicht der Komponente a1) um höchstens 100 g/mol vom zahlenmittleren Molekulargewicht der Komponente a2) und die OH-Funktionalität der Komponente a1) um höchstens 0,3 von der OH-Funktionalität der Komponente a2) ab.

Erfindungsgemäß handelt es sich bei der Komponente a3), sofern in der Polyolmischung enthalten, um ein oder mehrere vernetzend wirkende Verbindungen mit einem zahlenmittleren Molekulargewicht von 90 bis 400 g/mol. Bevorzugt sind dabei solche Vernetzer a3), die ein Molekulargewicht von 90 bis 300 g/mol, besonders bevorzugt von 150 bis 300 g/mol aufweisen.

Die erfindungsgemäßen Polyolmischungen können mit oder ohne Vernetzer a3) vorliegen. Sofern jedoch Vernetzer beispielsweise zur Modifizierung der mechanischen Eigenschaften eingesetzt werden, werden zweckmäßigerweise Vernetzer a3) mit einer OH-Funktionalität von 3 bis 8 eingesetzt. Vorzugsweise werden Vernetzer mit einer OH-Funktionalität von 3 bis 4 eingesetzt.

Der Gehalt an Alkaliionen im Vernetzer a3) ist in der Regel herstellungsbedingt und kann in einem weiten Bereich variieren. Als Vernetzer a3) sind grundsätzlich solche mit oder ohne Alkaliionen geeignet. In einer Ausführungsform weist der Vernetzer a3) einen Gehalt an Alkaliionen, vorzugsweise Kaliumionen, von bis zu 1200 ppm auf. Vorzugsweise beträgt der Gehalt an Alkaliionen, bevorzugt Kaliumionen, im Vernetzer a3) in dieser Ausführungsform bis zu 1000 ppm und insbesondere bevorzugt bis zu 600 ppm. Gemäß einer anderen Ausführungsform weist der Vernetzer a3) einen geringen Gehalt an Alkaliionen, vorzugsweise Kaliumionen, auf.

Als geeignete Vernetzer seien beispielhaft genannt: 3- und höherwertige Alkohole, wie z.B. Glycerin, Trimethylolpropan, Pentaerythrit, 2,2,6,6-Tetrahydroxymethyl-4-oxaheptandiol-1,7(Di-pentaerythrit), Tripentaerythrit, 3,3,7,7-Tetrahydroxymethyl-5-oxanonane(Di-trimethylolpropan) und Sorbit und die mit diesen Alkoholen gestarteten niedermolekularen Polyoxypropylen-, Polyoxyethylen- oder Polyoxypropylenpolyoxyethylen-polyole. Die Herstellung der alkoxylierten Alkohole kann nach den bereits oben genannten Verfahren erfolgen.

Die Vernetzer a3) können anstatt des Molekulargewichts auch mittels ihrer Hydroxylzahl charakterisiert werden. Wie dem Fachmann hinlänglich bekannt, kann eine Umrechnung von Molekulargewicht in Hydroxylzahl lediglich dann exakt erfolgen, wenn die OH-Funktionalität bekannt ist. Bevorzugt sind Vernetzer a3) mit einer Hydroxylzahl von 400 bis 5000 mg KOH/g, besonders bevorzugt solche mit einer Hydroxylzahl von 500 bis 5000 mg KOH/g und ganz besonders bevorzugt solche mit einer Hydroxylzahl von 500 bis 3000 mg KOH/g.

Als Vernetzer a3) besonders bevorzugt werden solche mit einem Molekulargewicht von 90 bis 300 g/mol, die eine OH-Funktionalität von 3 aufweisen. Ganz besonders bevorzugt werden als Vernetzer a3) mit Trimethylolpropan gestartetes Polyethylenoxid mit einem Molekulargewicht von 90 bis 300 g/mol.

Grundsätzlich können ein oder mehrere Vernetzer als Komponente a3) eingesetzt werden. Der Fachmann wählt Mischungen aus mehreren Vemetzem so, dass das erfindungsgemäße zahlenmittlere Molekulargewicht und gegebenenfalls eine bevorzugte OH-Funktionalität erreicht wird.

Die erfindungsgemäßen Polyolmischungen können neben den genannten Komponenten a1), a2) und gegebenenfalls a3) noch weitere Zusatzstoffe enthalten. Als Zusatzstoffe kommen beispielsweise Stabilisatoren, Füllstoffe und/oder Hilfsmittel in Betracht. Der Fachmann wählt die Zusatzstoffe gemäß den Erfordernissen der geplanten Anwendung. Der Einsatz der Stabilisatoren, Füllstoffe und Hilfsmittel erfolgt in Mengen, die für solche Zusatzstoffe üblich sind. Beispielsweise enthalten die erfindungsgemäßen Polyolmischungen im Falle einer Verwendung in Vergussmassen für Dialysefilter bevorzugt keine Füllstoffe.

Die erfindungsgemäßen Polyolmischungen weisen vielseitige Einsatzmöglichkeiten auf. Besonders bevorzugt ist hierbei die Umsetzung mit den weiter unten beschriebenen modifizierten isocyanaten. Hierdurch lassen sich erfindungsgemäße Vergussmassen herstellen.

### Vergussmassen

Eine Vergussmasse im Sinne dieser Erfindung ist eine Mischung aus mindestens zwei Reaktivkomponenten, die zum Vergießen geeignet ist und die in flüssiger oder viskoser Form in einen Körper eingebracht oder auf einen Körper aufgebracht wird und anschließend aushärtet. Bei einem solchen Körper kann es sich beispielsweise um eine Fläche, um ein Gefäß mit mindestens einer Öffnung oder um eine Form mit mindestens einer Vertiefung handeln. Die Begriffe Vergussmasse und Gießharz sollen äquivalent verstanden werden. Die Eigenschaften der Vergussmasse: Wasseraufnahme. Peressigsäuredesorption, Verschneidbarkeit, Nasssterilisierbarkeit, Migration von zytotoxischen Verbindungen beziehen sich auf den ausgehärteten Zustand.

Erfindungsgemäß enthalten die Vergussmassen Komponenten auf Basis (A) einer erfindungsgemäßen Polyolmischung und (B) mindestens eines modifizierten Isocyanats auf der Basis einer Isocyanat-Komponente b1) und einer Diol-Komponente b2).

Die Vergussmassen sind erhältlich durch Umsetzung einer erfindungsgemäßen Polyolmischung (A) mit mindestens einem modifizierten Isocyanat (B), das erhältlich ist durch Umsetzung einer Isocyanat-Komponente b1) mit einer Diol-Komponente b2), gegebenenfalls katalysiert durch einen Katalysator (C).

Das Einsatz-Verhältnis zwischen der Polyolmischung (A) und der Komponente (B) kann hierbei in einem weiten Bereich variieren. Bevorzugt werden A und B in solchen Mengen zur Reaktion gebracht, dass das Äquivalenzverhältnis von NCO-Gruppen der Komponente B zur Summe der reaktiven Wasserstoffatome der Komponente A 0,9:1 bis 1,3:1, vorzugsweise 0,95:1 bis 1,2:1 und besonders bevorzugt 1:1 bis 1,1:1 beträgt. Der Fachmann bestimmt die einzusetzenden Masseverhältnisse entsprechend.

Die erfindungsgemäßen Vergussmassen zeigen eine niedrige anfängliche Mischviskosität. Unter Mischviskosität ist diejenige Viskosität zu verstehen, die sich unmittelbar nach Mischen der Reaktivkomponenten einstellt. Durch eine geringe Mischviskosität wird das Ausfüllen von Formen möglich, bei denen eine komplexe Struktur vorliegt und die für die vollständige Ausfüllung eine niedrige Viskosität erfordern. Eine niedrige Viskosität ist beispielsweise beim Vergießen von Dialysefiltern mit einer Faserzahl größer 12000 vorteilhaft.

Die erfindungsgemäßen Vergussmassen zeigen unmittelbar nach erfolgter Mischung der reaktiven Komponenten (A), (B) und gegebenenfalls (C) üblicherweise eine Mischviskosität, welche bis zu 1500 mPa·s beträgt; bevorzugt ist eine Mischviskosität von bis zu 1000 mPa·s und besonders bevorzugt ist eine Mischviskosität von bis zu 600 mPa·s. Ganz besonders bevorzugt ist schließlich eine Mischviskosität von bis zu 500 mPa·s. Grundsätzlich ist eine möglichst geringe Mischviskosität erwünscht, da eine geringe Mischviskosität ein effizientes Vergießen ermöglicht. Aufgrund technischer Gegebenheiten ergibt sich jedoch andererseits eine praktische untere Grenze für die Mischviskosität. Beispielsweise kann die Mischviskosität im Bereich von 200 mPa·s oder höher liegen, insbesondere im Bereich von 250 bis 600 mPa·s.

### Modifiziertes Isocyanat (B)

Das modifizierte Isocyanat (B) ist erfindungsgemäß erhältlich durch Umsetzung einer Isocyanat-Komponente (b1) mit einer Diol-Komponente (b2), wobei Polyisocyanat-Prepolymere entstehen. Die Umsetzung erfolgt auf an sich bekannte Weise, indem unten beschriebene Isocyanat-Komponenten (b1), beispielsweise bei Temperaturen von etwa 80°C, mit unten beschriebenen Diol-Komponenten (b2) zu einem Polyisocyanat-Prepolymer zur Reaktion gebracht werden.

Im Rahmen der Erfindung ist es möglich, dass das modifizierte Isocyanat (B) noch weitere Zusatzstoffe enthält. Als Zusatzstoffe können beispielsweise Stabilisatoren, Füllstoffe und/oder Hilfsmittel eingesetzt werden. Der Fachmann setzt die genannten Zusatzstoffe nach den Erfordernissen des Anwendungsgebietes ein. Beispielsweise enthält die Komponente B in Vergussmassen für Dialysefilter bevorzugt keine Füllstoffe. Das modifizierte Isocyanat (B) enthält jedoch üblicherweise ein oder mehrere Hilfsmittel zur Reaktionskontrolle. Hierbei handelt es sich um Hilfsmittel, welche die Umsetzung der Komponenten (b1) und (b2) beeinflussen und/oder Nebenreaktionen bei der Reaktion von (b1) und (b2) und/oder während der späteren Lagerung nach erfolgter Umsetzung reduzieren. Bevorzugt enthält das modifizierte Isocyanat (B) von 0,1 bis 10 g eines Hilfsmittels zur Reaktionskontrolle pro 10 kg (B). Besonders bevorzugt enthält das modifizierte Isocyanat (B) von 0,2 bis 8 g eines Hilfsmittels zur Reaktionskontrolle pro 10 kg (B). Besonders bevorzugt kommen als Hilfsmittel zur Reaktionskontrolle Diolbis-chlorformiate, insbesondere Diethylenglykol-bis-chlorformiat oder Benzoylchlorid zum Einsatz.

Als Isocyanat-Komponente b1) kommen die üblichen aliphatischen, cycloaliphatischen und insbesondere aromatischen Di- und/oder Polyisocyanate oder deren Mischungen zum Einsatz. Insbesondere geeignet sind Diisocyanate, beispielsweise Toluylendiisocyanat (TDI). Bevorzugt werden Diphenylmethandiisocyanate (nachfolgend als MDI bezeichnet). Sofern MDI verwendet wird, können alle 2-Kern-Isomere (2,2'; 2,4' und 4,4') verwendet werden. Bevorzugt wird jedoch 4,4'-MDI eingesetzt.

Die Isocyanat-Komponente b1) kann zusätzlich modifiziert vorliegen, beispielsweise durch Einbau von Uretdion-, Carbamat-, Isocyanurat-, Carbodiimid-, Allophanat- und Urethangruppen.

Bevorzugt enthält die Komponente b1) 2 bis 10 Gew.-% eines Carbodiimidmodifizierten Isocyanates. Besoriders bevorzugt ist hierbei ein Carbodiimid-modifiziertes 4,4'-MDI. Ganz besonders bevorzugt enthält die Isocyanat-Komponente b1) 3 bis 7 Gew.-% Carbodiimid-modifiziertes 4,4'-MDI. Die angegebenen Zahlenwerte in Gew.-% Carbodiimid-modifiziertes Isocyanat beziehen sich auf ein Carbodiimid-modifiziertes Isocyanat, welches 10 Gew.-% Carbodiimid enthält. Bei abweichendem Carbodiimidgehalt rechnet der Fachmann die angegebenen Werte entsprechend um.

Als Diol-Komponente b2) kommen organische Polyhydroxyverbindungen mit einer OH-Funktionalität von 1,5 bis 2,5 zum Einsatz. Bevorzugt liegt die OH-Funktionalität im Bereich von 1,8 bis 2,2, besonders bevorzugt wird eine Diol-Verbindung mit einer OH-Funktionalität von 2 eingesetzt. Als Diol-Komponente b2) bevorzugt werden insbesondere alkoxylierte Diol-Verbindungen. Besonders bevorzugt werden als Diol-Komponente b2) Propylenglykole.

Zu den geeigneten Propylenglykolen zählen (Mono-)Propylenglykol und Dipropylenglykol sowie Oligo- und Polypropylenglykole, wobei letztere ausgehend von einer Diolverbindung durch Propoxylierung hergestellt werden können.

Erfindungsgemäß enthält die Diol-Komponente b2) eine Mischung aus mindestens zwei verschiedenen Propylenglykolen mit unterschiedlichem zahlenmittleren Molekulargewicht.

Bevorzugt enthält die Diol-Komponente b2) mindestens zwei verschiedene Propylenglykole b2x) und b2y) als Bestandteile mit unterschiedlichem Molekulargewicht, wobei als Bestandteil b2x) ein Propylenglykol mit einem Molekulargewicht von 700 bis 1300 g/mol und als Bestandteil b2y) ein Propylenglykol mit einem Molekulargewicht von 50 bis 200 g/mol eingesetzt wird.

Besonders bevorzugt enthält die Diol-Komponente (b2) eine Mischung, die mindestens drei unterschiedliche Propylenglykole b2x), b2y) und b2z) als Bestandteile mit unterschiedlichem Molekulargewicht enthält, wobei als Bestandteil b2x) ein Propylenglykol mit einem zahlenmittleren Molekulargewicht von 700 bis 1300 g/mol, als Bestandteil b2y) ein Propylenglykol mit einem zahlenmittleren Molekulargewicht von 250 bis 650 g/mol und als Bestandteil b2z) ein Propylenglykol mit einem zahlenmittleren Molekulargewicht von 50 bis 200 g/mol eingesetzt wird. Besonders bevorzugt als Bestandteil b2z) ist Dipropylenglykol.

Falls die Diol-Komponente b2) zwei unterschiedliche Propylenglykole b2x) und b2y) mit unterschiedlichem Molekulargewicht enthält, dann werden die beiden Bestandteile b2x) und b2y) bevorzugt im Mischungsverhältnis von 40 bis 60 Gew.-% b2x) und von 60 bis 40 Gew.-% b2y) eingesetzt. Besonders bevorzugt werden von 45 bis 55 Gew.-% b2x) und von 55 bis 45 Gew.-% b2y) eingesetzt, wobei die Summe aus b2x) und b2y) jeweils 100 Gew.-% ergibt.

Falls die Diol-Komponente b2) mindestens 3 unterschiedliche Propylenglykole gemäß der unter b2x), b2y) und b2z) genannten Eigenschaften enthält, dann werden die drei Komponenten b2x), b2y) und b2z) bevorzugt in folgendem Verhältnis eingesetzt: von 30 bis 40 Gew.-% b2x), von 30 bis 40 Gew.-% b2y) und von 20 bis 40 Gew.-% b2z). Besonders bevorzugt wird von 32 bis 36 Gew.-% b2x), von 35 bis 39 Gew.-% b2y) und von 25 bis 33 Gew.-% b2z) eingesetzt, wobei die Summe aus b2x), b2y) und b2z) jeweils 100 Gew.-% ergibt.

Bevorzugt hat das modifizierte Isocyanat (B) einen NCO-Gehalt von 18 bis 28 Gew.-%, besonders bevorzugt von 20 bis 25 Gew.-%.

Das modifizierte Isocyanat (B) weist außerdem eine Viskosität von 250 bis 1500 mPa·s auf; bevorzugt ist eine Viskosität von 250 bis 1000 mPa·s und besonders bevorzugt ist eine Viskosität von 250 bis 500 mPa·s.

Die beschriebenen modifizierten Isocyanate zeigen auch bei tiefen Temperaturen eine hohe Lagerstabilität und keine unerwünschte Kristallisation.

### Katalysator C

Die erfindungsgemäßen Vergussmassen können in Abwesenheit oder in Gegenwart von Katalysatoren hergestellt werden. Die Herstellung der Vergussmassen findet jedoch vorzugsweise in Gegenwart von Katalysatoren statt, welche die Reaktion des modifizierten Isocyanates (B) mit der Polyolmischung (A) stark beschleunigen.

Als Katalysatoren. (C) in Betracht kommen organische Metallverbindungen, vorzugsweise organische Zinnverbindungen, insbesondere die Zinn-(II)-salze von organischen Carbonsäuren, wie Zinn-(II)-diacetat, Zinn-(II)-dioctoat, Zinn-(II)-diethylhexoat und Zinn-(II)-dilaurat sowie die Dialkylzinn-(IV)-salze von organischen Carbonsäuren, wie z.B. Dibutylzinn-(IV)-diacetat, Dibutylzinn-(IV)-dilaurat, Dibutylzinn-(IV)-maleat und Di-octylzinn-(IV)-diacetat. Derartige Katalysatoren werden z.B. in der DE-A-3 048 529 beschrieben.

Als gut geeignet haben sich insbesondere Dialkylzinn-(IV)-mercaptoverbindungen erwiesen, wie Bislaurylzinn-(IV)-dimercaptid, sowie Verbindungen der allgemeinen Formeln R₂Sn(SR'-O-CO-R")₂ oder R₂Sn(SR'-CO-OR")₂, in denen R einen Alkylrest mit mindestens 8 Kohlenstoffatomen, R' einen Alkylenrest mit mindestens zwei Kohlenstoffatomen und R" einen Alkylrest mit mindestens vier Kohlenstoffatomen bedeuten. Als Katalysatoren dieser Art, die beispielsweise in der DD-A-218 668 beschrieben werden, seien beispielhaft genannt: Dioctylzinn-(IV)-bis(thioethylenglykol-2-ethylhexoat), Dioctylzinn-(IV)-bis(thioethylenglykollaurat), Dioctylzinn-(IV)-bis(thiolatoessigsäure-2-ethylhexylester, Dioctylzinn-(IV)-bis(thiolatoessigsäurehexylester) und Dioctylzinn-(IV)-bis(thiolatoessigsäurelaurylester).

Als Katalysatoren sehr gut bewährt haben sich ferner Organozinnverbindungen mit Zinn-Sauerstoff- oder Zinn-Schwefel-Bindungen, wie sie beispielsweise in der DD-A-255 535 beschrieben werden und die der allgemeinen Formeln (R₃Sn)₂O, R₂SnS, (R₃Sn)₂S, R₂Sn(SR')₂ oder RSn(SR')₃ entsprechen, wobei R und R' Alkylgruppen darstellen, die 4 bis 8 Kohlenstoffatome bei R sowie 4 bis 12 Kohlenstoffatome bei R' enthalten und R' außerdem -R"COOR"' und -R"OCOR'" bedeuten kann, in denen R" Alkylgruppen mit 1 bis 6 Kohlenstoffatomen und R"' Alkylengruppen mit 4 bis 12 Kohlenstoffatomen sind. Als Beispiele hierfür seien genannt: Bis(tributylzinn)oxid, Dibutylzinn-(IV)-sulfid, Dioctylzinn-(IV)-sulfid, Bis(tributylzinn)sulfid, Dibutylzinn-(IV)-bis(thioglykolsäure-2-ethylhexylester), Dioctylzinn-(IV)-bis(thioglykolsäure-2-ethylhexylester), Octylzinn-(IV)-tris(thioglykolsäure-2-ethyl-hexylester), Dioctylzinn-(IV)-bis(thioethylenglykol-2-ethyl-hexoat) und Dibutylzinn-(IV)-bis(thioethylenglykollaurat). Als Katalysatoren vorzugsweise verwendet werden Mono-n-octylzinn-(2-ethylhexylthioglykolat) und Di-n-octylzinn-bis-(2-ethylhexylthioglykolat). Besonders bevorzugt sind Katalysatoren (C) auf Basis von Dioctylzinn(IV)-dimercaptid.

Die Katalysatoren (C) können einzeln oder in Form von Katalysatorkombinationen eingesetzt werden.

Die vorliegende Erfindung umfasst sowohl die aus den beschriebenen Komponenten herstellbaren Vergussmassen als auch die Kombination der getrennt vorliegenden Komponenten aus (A) einer erfindungsgemäßen Polyolmischung und (B) einem oben beschriebenen modifizierten isocyanat zur gemeinsamen Anwendung.

Falls zur Herstellung der erfindungsgemäßen Vergussmassen Katalysatoren zum Einsatz kommen, dann werden diese bevorzugt mit der Polyolmischung (A) vermischt, welche schließlich mit der Komponente (B) vermischt und umgesetzt wird.

Die Katalysatoren werden üblicherweise in einer Menge von 0,001 bis 0,2 Gew.-Teilen, vorzugsweise 0,005 bis 0,015 Gew.-Teilen pro 100 Gew.-Teile der Polyolmischung (A) eingesetzt.

Erfindungsgemäß umfasst das Verfahren zur Herstellung der erfindungsgemäßen Vergussmassen das Mischen einer erfindungsgemäßen Polyolmischung (A), gegebenenfalls enthaltend ein Katalysator (C), mit mindestens einem modifizierten Isocyanat (B).

Das Mischen erfolgt dabei bevorzugt mittels einer Polyurethan-Zweikomponenten-Verarbeitungsmaschine. Die sich unmittelbar nach erfolgter Mischung einstellende Mischviskosität liegt im erfindungsgemäßen Verfahren bevorzugt im weiter oben definierten Bereich. Anschließend werden die Vergussmassen vergossen und der Aushärtung unterzogen.

Unter Vergießen soll jede Maßnahme verstanden werden, die der anfänglich fließfähigen Vergussmasse diejenige Gestalt gibt, die sie nach dem Aushärten hat. Unter Vergießen soll insbesondere das Einbringen in oder das Aufbringen auf einen Körper verstanden werden. Bei einem solchen Körper kann es sich beispielsweise um eine Fläche, ein Rahmen, ein Gefäß mit mindestens einer Öffnung oder um eine Form mit mindestens einer Vertiefung handeln. Die Vergussmasse kann grundsätzlich in Kontakt mit dem Körper bleiben oder aus diesem herausgelöst werden. Bevorzugt wird die Vergussmasse nach erfolgter Aushärtung nicht von der Form getrennt, sondern bildet mit dieser eine Einheit.

Die Vorteile des Verfahrens zeigen sich insbesondere beim Vergießen in eine komplexe Form, die mehrere Ecken und/oder Kanten enthält, die von der Vergussmasse umschlossen werden sollen.

Die Aushärtung kann grundsätzlich in einem oder in mehreren Schritten erfolgen, welche sich in den Umgebungsbedingungen, insbesondere der Temperatur, unterscheiden. Beispielsweise kann die Aushärtung in einem Vorhärtungsschritt und einem Nachhärtungsschritt erfolgen. Bevorzugt ist jedoch die Aushärtung in einem Schritt.

Die Aushärtung erfolgt im allgemeinen ohne weiteres Zutun durch Reaktion der NCO-Gruppen mit den reaktiven Wasserstoffatomen, insbesondere der OH-Gruppen. Gegebenenfalls wird die Temperatur und die Atmosphäre der Umgebung beim Aushärtungsschritt kontrolliert und/oder gesteuert. Während der Aushärtung läuft im allgemeinen eine chemische Vernetzungsreaktion ab. Die Aushärtung ist abgeschlossen, sobald die Vergussmasse weitgehend ihre endgültigen Eigenschaften, insbesondere ihre endgültige Härte erreicht hat.

Die Aushärtung erfolgt üblicherweise in einem Zeitraum von Minuten bis mehrere Stunden, beispielsweise von 0,3 bis 4 Stunden, bevorzugt in einem Zeitraum von 1 bis 3 Stunden.

Die erfindungsgemäßen Vergussmassen zeigen nach erfolgter Aushärtung im allgemeinen eine Härte von 50 bis 70 Grad Shore-D. Bevorzugt weisen die erfindungsgemäßen Vergussmassen jedoch eine Härte von 55 bis 65 Grad Shore-D auf. Besonders bevorzugt, beispielsweise für Anwendungen als Vergussmasse in Dialysefiltern, ist eine Härte von 58 bis 62 Grad Shore-D. Die Härte in Grad Shore-D bezieht sich auf DIN 53505 bei einer Temperatur von 23°C. Der Fachmann wählt die Zusammensetzung der Vergussmassen, beispielsweise Art und Menge des Vernetzers a3), entsprechend.

Je nach Anwendungsgebiet können die Vergussmassen erst nach einem Reinigungsschritt, zum Beispiel einem Sterilisationsschritt im Falle von Vergussmassen in Dialysefiltern, einsatzbereit sein.

Die erfindungsgemäßen Vergussmassen haben vielfältige Einsatzmöglichkeiten. Zu den möglichen Anwendungen zählt der Einsatz als Formmasse, z. B. im Formenbau oder bei der Herstellung von Prototypen, und als Einbettungswerkstoff, z. B. als Vergussmasse für Bauteile im Elektro- und Elektronikbereich oder in Medizinprodukten. Bevorzugt ist die Verwendung als Einbettungswerkstoff. Insbesondere kommen die erfindungsgemäßen Vergussmassen als Einbettungswerkstoff im Elektro- oder Elektronikbereich sowie bei der Filtration wässriger Medien zum Einsatz. Insbesondere bevorzugt sind medizinisch-technische Anwendungen. Ganz besonders bevorzugt ist die Verwendung der Vergussmassen zur Einbettung von Hohlfasern, insbesondere in Dialysefiltern.

### Dialysefilter

Ein sogenannter Dialysefilter oder Dialysator stellt jene Komponente in einem Dialysegerät dar, das die Austauschmembran enthält, an welcher der Stoffaustausch bei der Blutwäsche vorgenommen wird. Überwiegend kommen sogenannte Kapillardialysatoren zum Einsatz, auf die sich die in der vorliegenden Erfindung genannten Dialysefilter beziehen. Die Dialysefilter bestehen aus einem Bündel von Hohlfasern, welches üblicherweise 10000 bis 15000 Fasern enthält, und welches jeweils an zwei Enden eines Hohlkörpers in eine Matrix einer Vergussmasse eingebettet ist. Der Hohlkörper besteht üblicherweise aus einem transparenten Kunststoff, beispielsweise Polycarbonat, und wird derart in ein Dialysatorgehäuse eingebaut, dass Blut durch das Innere der Hohlfasern geleitet werden kann. Im Hohlkörper des Dialysefilters umspült die Dialysierflüssigkeit die blutdurchströmten Hohlfasern. Die Wand der Hohlfasern bildet die eigentliche Filtermembran, an welcher der Stoffaustausch während der Dialysebehandlung erfolgt.

Erfindungsgemäß umfasst das Verfahren zur Herstellung der erfindungsgemäßen Dialysefilter das Mischen einer erfindungsgemäßen Polyolmischung (A), gegebenenfalls enthaltend ein Katalysator (C), mit einem modifizierten Isocyanat (B).

Das Mischen der beschriebenen Komponenten erfolgt bevorzugt mittels einer Polyurethan-Zweikomponenten-Verarbeitungsmaschine. Die sich nach erfolgter Mischung unmittelbar einstellende Mischviskosität liegt im erfindungsgemäßen Verfahren bevorzugt im Bereich von 250 bis 600 mPa·s; besonders bevorzugt liegt sie im Bereich von 300 bis 500 mPa·s. Das Reaktionsgemisch wird anschließend in einer dosierten Menge in die Hohlfasern enthaltende Form eingetragen.

Der Eintrag der Vergussmasse erfolgt hierbei bevorzugt in einen in einer Zentrifuge rotierenden Hohlkörper, welcher Hohlfasern enthält, wobei der Hohlköper eine Vorstufe eines Dialysefilters ist. Durch Zentrifugalkraft wird das flüssige Reaktionsgemisch an die jeweiligen beiden Enden des Dialysefilters unter Umschließung der Hohlfasern transportiert und härtet zum kompakten, im wesentlichen klaren Verguss aus.

Der Aushärtungsschritt erfolgt wiederum ohne weiteres Zutun durch Reaktion der NCO-Gruppen mit reaktiven Wasserstoffatomen, insbesondere der OH-Gruppen. Der Aushärtungsschritt ist abgeschlossen, sobald die Vergussmasse weitgehend ihre endgültigen Eigenschaften, insbesondere ihre Härte und ihre Stabilität bei der Nasssterilisation und das Ausbleiben der Migration zytotoxischer Verbindungen erreicht hat.

Durch einen anschließenden Schneidprozess werden üblicherweise die Öffnungen der Hohlfasern freigelegt. Der Dialysefilter ist im allgemeinen nach einem Reinigungs- und Sterilisationsprozess einsatzbereit.

Durch das erfindungsgemäße Verfahren ist es möglich, Vergussmassen herzustellen, die heissdampfsterilisierbar und nicht zytotoxisch sind und so im medizinisch-technischen Bereich eingesetzt werden können, und die gleichzeitig komplexe Strukturen, beispielsweise eine hohe Faserzahl in einem Dialysefilter, vollständig umschließen.

Die ausgehärteten Vergussmassen sind resistent gegenüber Desinfektionsmitteln. Insbesondere zeigen die erfindungsgemäßen Vergussmassen eine geringe Aufnahme von Wasserdampf oder kochend heißem Wasser. Die erfindungsgemäßen Vergussmassen lassen sich über einen Zeitraum von zwei Wochen ohne Bildung von Feinstaub schneiden, der andernfalls die Poren verstopfen kann. Die erfindungsgemäßen, gehärteten Vergussmassen sind transparent, nicht zytotoxisch und besitzen eine verbesserte Haftung zu anderen Werkstoffen, z.B. Polycarbonaten, bei erhöhten Temperaturen über einen längeren Zeitraum. Die Vergussmassen sind gegen Percarbonsäuren stabil, so dass Formkörper aus solchen Vergussmassen mit Peressigsäure sterilisiert werden können. Die erfindungsgemäßen Vergussmassen zeigen eine hohe Hydrophobie und eine ausreichende Vernetzungsdichte.

Die noch fließfähigen Vergussmassen lassen sich auch ohne Schaumbildung vergießen. Gleichzeitig zeigen die Vergussmassen unmittelbar nach Mischung der Reaktivkomponenten eine niedrige Mischviskosität. Die Vergussmassen sind bereits nach 2 Stunden schneidbar, härten jedoch nicht so stark nach, so dass sie auch nach mehr als 24 Stunden noch schneidfähig sind. Vorteilhaft ist ferner, dass die erfindungsgemäßen Vergussmassen auf Basis von Polyurethan mit allen üblichen Hohlfaserarten, wie z.B. Cuprophan-, Polysulfon-, Polycarbonat- oder Cellulosefasern verarbeitbar sind und die Polycarbonate vor der Verarbeitung keiner Vorbehandlung durch CoronaEntladung zur Verbesserung der Haftfestigkeit bedürfen.

Die erfindungsgemäßen Polyolmischungen führen somit in Kombination mit geeigneten modifizierten Isocyanaten zu Vergussmassen mit den vorgenannten Vorteilen und ermöglichen durch die geringe resultierende Mischviskosität das zeiteffiziente Vergießen komplexer Strukturen.

### Beispiele

### 1. Bestimmung der Kenngrößen

Die Viskosität wurde nach DIN 53018 bei einer Temperatur von 25°C mit einem Rotationsviskosimeter der Firma Haake bestimmt (Messeinrichtung Platte/Kegel). Die Mischviskosität wurde rechnerisch bestimmt, da sie sich auf den Zeitpunkt Null, das heißt unmittelbar vor Beginn der Reaktion bezieht. Es wurde die folgende Formel verwendet: log(Mischviskosität) = {Masseanteil Komponente (A) * log(Viskosität (A)) + Masseanteil Komponente (B) * log(Viskosität (B)}, wobei die Summe der Masseanteile aus Komponente (A) und (B) eins ergibt.

Die Wasseraufnahme wurde bestimmt, indem vorher gewogene, runde Prüfkörper mit 68 mm Durchmesser und 5 mm Dicke 5 Stunden in einem Gefäß mit Wasser gekocht werden. Anschließend wurde die Masse erneut bestimmt und die prozentuale Gewichtszunahme ermittelt.

Die Peressigsäuredesorption wurde bestimmt, indem 5 g der Vergussmasse in Form mehrerer 1 mm dicker, kreisförmiger Probenstücke mit einem Durchmesser von 35 mm 2 Stunden bei 20°C in 100 ml einer wässrigen Lösung, welche 3,5 Gew.-% Peressigsäure und 26 Gew.-% Wasserstoffperoxid enthält, gelagert wurden. Anschließend wurde das Probenstück einmal mit destilliertem Wasser abgespült und in 100 ml destilliertem Wasser unter regelmäßigem Rühren gelagert. Nach vier Stunden Lagerung wurde die herausgelöste Menge an Peroxoverbindung iodometrisch durch Rücktitration von oxidiertem Iodid mit einer 0,01-molaren Thiosulfatlösung bestimmt.

Die OH-Funktionalität wurde rechnerisch nach der Formel Hydroxylzahl [in mg/g KOH] * zahlenmittleres Molekulargewicht / 56100 = OH-Funktionalität bestimmt.

Die Härte wurde nach Shore-D gemäß DIN 53505 bestimmt (Temperatur 23°C).

### 2. Einsatzstoffe

**Tabelle 1 - Polyolmischung (A)**

| | | |
|---|---|---|
| Fettbasiertes Polyol a1) | a1-1) | Rizinusöl gemäß DAB-10 |
| Polyetherol a2) | a2-1) | Trimethylol-gestartetes Polyoxypropylen MG = 1040 g/mol |
| | a2-V2) | Polyetherpolyol auf Basis Saccharose/ Pentandiol/ Di-ethylenglykol/ Polyoxypropylen mit MG = 450 g/mol und OH-Funktionalität 4 |
| | a2-V3) | Glycerin-gestartetes Polyetherpolyol auf Basis von Ethylenoxid und Propylenoxid MG = 3550 g/mol und OH-Funktionalität 2,6 |
| Vernetzer a3) | a3-1) | Trimethylol-gestartetes Polyoxyethylen MG = 180 g/mol |
| | a3-2) | Trimethylol-gestartetes Polyoxypropylen MG = 200 g/mol |
| Katalysator C) | Dioctylzinn(IV)-dimercaptid | |

| | | |
|---|---|---|
| MG = Molekulargewicht | | |

**Tabelle 2 -Modifiziertes Isocyanat (B)**

| | | |
|---|---|---|
| Isocyanat-Komponente b1) | b1-1) | 4,4'-MDI |
| | b1-2) | Carbodiimid-modifiziertes 4,4'-MDI mit einem Carbodiimid-Gehalt von 10 Gew.-% |
| Diol-Komponente b2) | b2-1) | Propylenglykol mit MG = 1080 g/mol |
| | b2-2) | Propylenglykol mit MG = 450 g/mol |
| | b2-3) | Dipropylenglykol |

| | | |
|---|---|---|
| MG = Molekulargewicht | | |

### 3. Herstellung der Komponenten

### Polyolmischung

Aus a1), a2), a3) und (C) wurde durch Vermischen der in den Tabellen 1 und 3 angegebenen Komponenten und Gewichtsverhältnisse unter Rühren bei Raumtemperatur jeweils 10 kg der Polyolmischung (A) hergestellt.

### Modifiziertes Isocyanat

Die Komponente b1-1) wurde in einem Laborrührreaktor mit Heiz- und Kühlvorrichtung vorgelegt. Dazu wurde gegebenenfalls die Komponente b1-2) zugegeben und beide Isocyanate vermischt. Aus den Komponenten b2-1), b2-2) und b2-3) wurde ein Gemisch hergestellt, dem noch 0,7g pro 10kg modifiziertem Isocyanat Diglykol-bis-chlorformiat zugesetzt wurde. Das Glykolgemisch wurde dabei langsam und unter Rühren dem Isocyanat zugesetzt und die einsetzende Reaktion der NCO-Gruppen mit den reaktiven Wasserstoffatomen so gesteuert, dass der Umsatz des Glykolgemisches mit dem im Überschuss vorhandenen Isocyanat bei 80°C im Zeitraum von 60 min erfolgte, worauf eine Abkühlphase folgte.

### 4. Herstellung der Vergussmassen und Dialysefilter

Die beschriebenen Komponenten wurden im in der Tabelle 4 angegebenen Mischungsverhältnis mittels einer Polyurethan-Zweikomponenten-Verarbeitungsmaschine gemischt und das Reaktionsgemisch in der genau dosierten Menge in den mit Hohlfasern gefüllten rotierenden Dialysefilter eingetragen.

### 5. Zusammensetzungen

**Tabelle 3 - Zusammensetzung der Polyolmischungen**

| Beispiel | Polyol a1) | Polyetherol a2) | | | Vernetzer a3) | |
|---|---|---|---|---|---|---|
| | a1-1) | a2-1) | a2-V2) | a2-V3) | a3-1) | a3-2) |
| | Anteil in Gew.-% in (A) | | | | | |
| 1 | 56,4 | 37,6 | 0 | 0 | 6 | 0 |
| 2V | 94 | 0 | 0 | 0 | 6 | 0 |
| 3V | 0 | 75 | 25 | 0 | 0 | 0 |
| 4V | 79 | 0 | 21 | 0 | 0 | 0 |
| 5V | 43 | 0 | 0 | 43 | 0 | 14 |

**Tabelle 4 - Zusammensetzung der Vergussmassen**

| Beispiel | Polyolmischung (A) | Katalysator (C) | Masseverhältnis A:B | Isocyanat-komponente b1) | | Diol-Komponente b2) | | |
|---|---|---|---|---|---|---|---|---|
| | | | | b1-1) | b1-2) | b2-1) | b2-2) | b2-3) |
| | gemäß Beispiel | Zugabe in g pro kg (A) | | Anteil Gew.-% in (B) | | | | |
| 6 | 1 | 0,6 | 100:72 | 79 | 4 | 5,8 | 6,3 | 4,9 |
| 7V | 2V | 0,6 | 100:70 | 87 | 0 | 0 | 4,9 | 8,1 |
| 8V | 3V | 0,8 | 100:86 | 67,5 | 4 | 20 | 8,5 | 0 |
| 9V | 4V | 0,8 | 100:72 | 87 | 0 | 0 | 4,9 | 8,1 |
| 10V | 5V | 0,8 | 100:72 | 87 | 0 | 0 | 4,9 | 8,1 |

**Tabelle 5: Vergleich der Eigenschaften der Vergussmassen**

| Beispiel | Viskosität (A) | Viskosität (B) | Mischviskosität | Wasser-Aufnahme | Härte | Peressigsäure-Desorption |
|---|---|---|---|---|---|---|
| | [mPa·s] | | | [Gew.-%] | [Grad Shore-D] | [ppm] |
| 6 | 535 | 360 | 454 | + 0,97 | 61 | 69,5 |
| 7V | 820 | 650 | 745 | + 0,77 | 60 | 29,8 |
| 8V | 450 | 350 | 400 | + 1,96 | 60 | 268,1 |
| 9V | 787 | 650 | 727 | + 0,81 | 72 | n.b. |
| 10V | 863 | 650 | 767 | + 1,82 | 58 | n.b. |

| | | | | | | |
|---|---|---|---|---|---|---|
| n.b.: nicht bestimmt | | | | | | |

## Patentansprüche

1. Polyolmischung, enthaltend als Komponenten
a1) mindestens ein fettbasiertes Polyol,
a2) mindestens ein Polyetherol mit einem zahlenmittleren Molekulargewicht von 500 bis 2000 g/mol und
a3) ein oder mehrere Vernetzer mit einem zahlenmittleren Molekulargewicht von 90 bis 400 g/mol,
**dadurch gekennzeichnet, dass**
x) das zahlenmittlere Molekulargewicht der Komponente a1) um höchstens 400 g/mol vom zahlenmittleren Molekulargewicht der Komponente a2) abweicht und
xi) die OH-Funktionalität der Komponenten a1) und a2) um nicht mehr als 0,5 voneinander abweicht,
wobei die Polyolmischung, bezogen auf das Gesamtgewicht von a1), a2) und a3), von 45 bis 65 Gew.-% der Komponente a1), von 34 bis 54 Gew.-% der Komponente a2) und von 1 bis 21 Gew.-% der Komponente a3) enthält.

2. Polyolmischung nach Anspruch 1, **dadurch gekennzeichnet, dass** die Komponente a1) mindestens ein Polyol auf Basis von Rizinusöl enthält.

3. Polyolmischung nach den Ansprüchen 1 oder 2, **dadurch gekennzeichnet, dass** die Komponenten a1) und a2) jeweils eine OH-Funktionalität von 2,6 bis 3 aufweisen.

4. Polyolmischung nach den Ansprüchen 1 bis 3, **dadurch gekennzeichnet, dass** die Komponente a2) als Polyetherol propoxyliertes Trimethylolpropan und/oder propoxyliertes Glycerin enthält.

5. Vergussmasse enthaltend Komponenten auf der Basis
(A) einer Polyalmischung gemäß den Ansprüchen 1 bis 4 und
(B) mindestens eines modifizierten Isocyanats auf der Basis einer Isocyanat-Komponente b1) und einer Diol-Komponente b2).

6. Vergussmasse nach Anspruch 5, **dadurch gekennzeichnet, dass** die Diol-Komponente b2) eine Mischung aus mindestens zwei verschiedenen Propylenglykolen mit unterschiedlichem zahlenmittleren Molekulargewicht ist.

7. Vergussmasse nach den Ansprüchen 5 oder 6, **dadurch gekennzeichnet, dass** die Diol-Komponente b2) eine Mischung ist, die mindestens drei unterschiedliche Propylenglykole mit unterschiedlichem Molekulargewicht enthält, wobei mindestens eines der Propylenglykole ein zahlenmittleres Molekulargewicht von 700 bis 1300 g/mol, mindestens eines der Propylenglykole ein zahlenmittleres Molekulargewicht von 250 bis 650 g/mol und mindestens eines der Propylenglykol ein zahlenmittleres Molekulargewicht von 50 bis 200 g/mol aufweist.

8. Verwendung von Vergussmassen gemäß den Ansprüchen 5 bis 7 als Einbettungswerkstoff.

9. Verwendung von Vergussmassen nach Anspruch 8 als Einbettungswerkstoff in Dialysefiltem.

10. Verfahren zur Herstellung von Vergussmassen gemäß den Ansprüchen 5 bis 9, umfassend das Mischen
(A) einer Polyolmischung gemäß den Ansprüchen 1 bis 4, gegebenenfalls enthaltend ein Katalysator (C), mit
(B) mindestens einem modifizierten Isocyanat auf der Basis einer Isocyanat-Komponente b1) und einer Diol-Komponente b2).

11. Verfahren zur Herstellung von Vergussmassen nach Anspruch 10, **dadurch gekennzeichnet, dass** sich eine Mischviskosität einstellt, die bis zu 600 mPa·s beträgt.

12. Dialysefilter, umfassend eine Vergussmasse gemäß den Ansprüchen 5 bis 7 oder hergestellt mittels eines Verfahrens gemäß den Ansprüchen 10 oder 11.

13. Kombination umfassend als getrennt vorliegende Komponenten
(A) eine Polyolmischung gemäß den Ansprüchen 1 bis 4 und
(B) mindestens ein modifiziertes Isocyanat auf der Basis einer Isocyanat-Komponente b1) und einer Diol-Komponente b2)
zur gemeinsamen Anwendung.

## Claims

1. A polyol mixture which comprises as components
a1) at least one fat-based polyol,
a2) at least one polyetherol having a number average molecular weight of from 500 to 2000 g/mol and
a3) one or more crosslinkers having a number average molecular weight of from 90 to 400 g/mol,
wherein
x) the number average molecular weight of component a1) differs by not more than 400 g/mol from the number average molecular weight of component a2) and
xi) components a1) and a2) do not differ from one another in OH functionality by more than 0.5,
where the polyol mixture, based the total weight of a1), a2) and a3), comprises from 45 to 65% by weight of component a1), from 34 to 54% by weight of component a2) and from 1 to 21% by weight of component a3).

2. The polyol mixture according to claim 1, wherein component a1) comprises at least one polyol based on castor oil.

3. The polyol mixture according to claims 1 or 2, wherein components a1) and a2) each have an OH functionality of from 2.6 to 3.

4. The polyol mixture according to claims 1 to 3, wherein component a2) comprises propoxylated trimethylolpropane and/or propoxylated glycerol as polyetherol.

5. An embedding composition comprising components based on
(A) a polyol mixture according to claims 1 to 4 and
(B) at least one modified isocyanate based on an isocyanate component b1) and a diol component b2).

6. The embedding composition according to claim 5, wherein the diol component b2) is a mixture of at least two different propylene glycols differing in number average molecular weight.

7. The embedding composition according to claims 5 or 6, wherein the diol component b2) is a mixture which comprises at least three different propylene glycols differing in molecular weight, where at least one of the propylene glycols has a number average molecular weight of from 700 to 1300 g/mol, at least one of the propylene glycols has a number average molecular weight of from 250 to 650 g/mol and at least one of the propylene glycols has a number average molecular weight of from 50 to 200 g/mol.

8. The use of embedding compositions according to claims 5 to 7 as potting material.

9. The use of embedding compositions according to claim 8 as potting material in dialysis filters.

10. A process for producing embedding compositions according to claims 5 to 9, comprising the mixing of
(A) a polyol mixture according to claims 1 to 4, if appropriate comprising a catalyst (C), with
(B) at least one modified isocyanate based on an isocyanate component b1) and a diol component b2).

11. The process for producing embedding compositions according to claim 10, wherein a mixed viscosity which is up to 600 mPa·s is set up.

12. A dialysis filter comprising an embedding composition according to claims 5 to 7 or produced by a process according to claims 10 or 11.

13. A combination comprising as components which are present separately
(A) a polyol mixture according to claims 1 to 4 and
(B) at least one modified isocyanate based on an isocyanate component b1) and a diol component b2)
but are to be used together.

## Revendications

1. Mélange de polyols, contenant comme composants
a1) au moins un polyol à base de graisse,
a2) au moins un polyétherol ayant une masse moléculaire moyenne en nombre de 500 à 2 000 g/mole et
a3) un ou plusieurs agents de réticulation ayant une masse moléculaire moyenne en nombre de 90 à 400 g/mole,
**caractérisé en ce que**
x) la masse moléculaire moyenne en nombre du composant a1) diffère d'au maximum 400 g/mole de la masse moléculaire moyenne en nombre du composant a2) et
xi) les fonctionnalités OH des composants a1 et a2) ne diffèrent pas l'une de l'autre de plus de 0,5,
le mélange de polyols contenant, par rapport au poids total de a1), a2) et a3), de 45 à 65 % en poids du composant a1), de 34 à 54 % en poids du composant a2) et de 1 à 21 % en poids du composant a3).

2. Mélange de polyols selon la revendication 1, **caractérisé en ce que** le composant a1) contient au moins un polyol à base d'huile de ricin.

3. Mélange de polyols selon la revendication 1 ou 2, **caractérisé en ce que** les composants a1) et a2) présentent chacun une fonctionnalité OH de 2,6 à 3.

4. Mélange de polyols selon les revendications 1 à 3, **caractérisé en ce que** le composant a2) contient en tant que polyétherol du triméthylolpropane propoxylé et/ou du glycérol propoxylé.

5. Matière à couler contenant des composants à base
(A) d'un mélange de polyols selon les revendications 1 à 4 et
(B) d'au moins un isocyanate modifié à base d'un composant isocyanate b1) et d'un composant diol b2).

6. Matière à couler selon la revendication 5, **caractérisée en ce que** le composant diol b2) est un mélange d'au moins deux propylèneglycols différents ayant différentes masses moléculaires moyennes en nombre.

7. Matière à couler selon la revendication 5 ou 6, **caractérisée en ce que** le composant diol b2) est un mélange qui contient au moins trois propylèneglycols différents ayant des masses moléculaires différentes, au moins l'un des propylèneglycols ayant une masse moléculaire moyenne en nombre de 700 à 1 300 g/mole, au moins l'un des propylèneglycols ayant une masse moléculaire moyenne en nombre de 250 à 650 g/mole et au moins l'un des propylèneglycols ayant une masse moléculaire moyenne en nombre de 50 à 200 g/mole.

8. Utilisation des matières à couler selon les revendications 5 à 7 en tant que matériau de scellement.

9. Utilisation des matières à couler selon la revendication 8, en tant que matériau de scellement dans des filtres à dialyse.

10. Procédé pour la préparation de matières à couler selon les revendications 5 à 9, comprenant le mélange
(A) d'un mélange de polyols selon les revendications 1 à 4, contenant éventuellement un catalyseur (C), avec
(B) au moins un isocyanate modifié à base d'un composant isocyanate b1) et d'un composant diol b2).

11. Procédé pour la préparation de matières à couler selon la revendication 10, **caractérisé en ce qu'**on ajuste la viscosité du mélange jusqu'à 600 mPa.s.

12. Filtre à dialyse, comprenant une matière à couler selon les revendications 5 à 7 ou préparée par un procédé selon la revendication 10 ou 11.

13. Association comprenant en tant que composants présents séparément
(A) un mélange de polyols selon les revendications 1 à 4 et
(B) au moins un isocyanate modifié à base d'un composant isocyanate b1) et d'un composant diol b2)
pour l'utilisation simultanée.
